# EUROPEAN PATENT APPLICATION

(11) **EP 4 379 064 A1**
(43) Date of publication of application: **05.06.2024**
(21) Application number: 22210144.6
(22) Date of filing: 29.11.2022
(51) Int. Cl.: C12Q 1/6886

(54) **DIAGNOSTIC METHODS AND DIAGNOSTIC ASSAY COMPRISING DETECTION OF LNCRNAS**

(71) Applicant: Universität Heidelberg, 69117 Heidelberg (DE); NewGiza University, 12577 Giza (EG)
(72) Inventor: Breuhahn, Kai, 69123 Heidelberg (DE); Rose, Fabian, 69115 Heidelberg (DE); Abdelaziz Fahmy, Ahmed, Cairo (EG); Magdy El-Ekiaby, Nada, 12577 Giza (EG); Schirmacher, Peter, 69118 Heidelberg (DE)
(74) Representative: Cohausz & Florack

(57) **Abstract**

The present application provides a diagnostic assay or kit and a method for identifying,
detecting, and/or monitoring a disease, wherein the method comprises providing a sample from a subject and determining the expression level of one or more long non-coding RNAs (lncRNAs) selected from the group consisting of CYTOR, SNHG17, MIR4435-2HG, and SNHG1 in the sample; wherein an overexpression of these one or more long non-coding RNAs in the sample indicates the disease.

## Description

### TECHNICAL FIELD OF THE INVENTION

The present invention relates to the expression levels of certain long non-coding RNAs (lncRNAs) and methods for identifying, detecting, and/or monitoring a disease therewith, and for identifying whether a subject would benefit from a certain treatment for the disease, and/or if a treatment for a disease is successful or not, and/or if a disease relapses. The invention further relates to a diagnostic assay or kit that is suitable for carrying out at least one of the methods.

### BACKGROUND OF THE INVENTION

Detection of early disease stages such as premalignant conditions, the presence of tumors and their sensitivity to drugs, as well as tracing therapeutic success is a medical but also socio-economic problem for patients and clinicians. Patients suffer from uncertainty and psychic stress during frequent medical check-ups e.g., due to familial genetic risk factors or follow-up examinations altering therapy and recovery. In clinics, stratification of patients for targeted therapies is usually difficult due to the lack of rapid, reliable, and easy read-outs. Monitoring therapeutic success, e.g., in cancer treatment, is time-consuming, often requires expensive equipment and is a stressful situation for patients.

Earliest stages of chronic diseases, which usually form the basis for disease progression and in particular cancer development, are difficult to detect In many cases, disease biomarkers that are suitable to identify patients eligible for specific therapies, especially in the stage of precancerous condition, after tumor initiation and during tumor progression, are missing or insufficient.

Therefore, there still is an unmet need for the robust detection of biomarkers, in particular such that define earliest disease stages, disease progression, and therapeutic success. The methods for detecting the biomarkers in patients should ideally be reliable, cost-efficient, and risk-free.

In the field of cancer diagnostics, it is known to histo-pathologically evaluate tissue specimen isolated from patients, e.g., by needle biopsy. This method has the disadvantage that the classification of diseases is complicated due to the heterogeneity of the isolated tissues and due to the remaining degree of subjectivity in histological analyses. In addition, the identification of cancer subtypes eligible for specific treatment is usually difficult based on histo-morphological characteristics. Lastly, needle biopsies, aspiration cytology, or surgical intervention are not risk-free and may cause severe side effects, such as pain, bleeding, stress, and tumor cell dissemination.

In some areas it has become possible to identify cancer subtypes by molecular diagnostics, e.g., via the detection of gene-specific mutations or genomic alterations. This allows identifying patients whose tumor bears one or more specific mutations, thereby rendering the patients eligible for targeted therapy by using e.g., mutation/alteration-specific drugs. However, molecular diagnosis and targeted therapy is generally only beneficial for the fraction of patients that is eligible for one of the available targeted drug treatments, whereas patients with different or unknown genetic alterations and/or signaling pathway activation do not benefit from these drugs.

Moreover, it is known in the field to analyse tumors by measurement of tumor markers in body fluids, e.g. by measuring alpha-fetoprotein (AFP) to detect liver cancer, or by measuring prostate-specific antigen (PSA) to detect prostate cancer. However, many established tumor markers are not reliable: For instance, only about 25% of patients who received a prostate biopsy due to increasing PSA levels are found to have cancer at the time of biopsy.

In addition to the aforementioned approaches, liquid biopsy has been suggested to improve cancer diagnostics. Liquid biopsy is a minimally invasive and risk-free method to acquire patient material, e.g. by drawing and analysing blood. However, the detection of biomarkers in serum or plasma as proxy for diseased tissues is difficult due to dilution effects in blood. In addition, previously-known diagnostic methods employing liquid biopsy are hampered by low reproducibility, thus preventing clinical application.

Previously published studies for the detection of potential liquid biopsy markers are based on the direct comparison between liquid biopsy samples derived from cancer patients and those derived from healthy controls. One reason for low reproducibility of liquid biopsy-based methods is assumed to be that information/signals from tumor cells have comparably low impact in light of the information/signals originating from non-tumor cells, which are also present and thus equally measured in the serum derived from the patients.

Known liquid biopsy approaches focus e.g. on circulating tumor cells (CSCs), cell-free tumor DNA (cfDNA or ctDNA), or non-coding RNA (ncRNA). The group of ncRNA comprises e.g., microRNA (miRNA) and long non-coding RNA (lncRNA).

It is known that IncRNAs can be dysregulated in tumors and that IncRNAs can act as tumor suppressors as well as oncogenes. LncRNAs are defined as RNA transcripts larger than 200 nt that do not appear to have protein-coding potential.

Yan et al., Cancer Cell 28:529 (2015) describe an siRNA screening strategy to identify cancer driver IncRNAs.

Liang et al., Nature Communications 13:2996 (2022) describe a nine-lncRNA signature for predicting distant metastasis in locoregionally advanced nasopharyngeal carcinoma.

Li et al., Front Oncol. 10:780 (2020) describe an eleven-lncRNA signature indicative of a liver cancer prognosis. lncRNA was isolated from tumor tissue.

Zhu etal., Theranostics, 11(1):181 (2021) describe a signature of three extracellular RNAs as potential biomarker for liver cancer. The extracellular RNAs comprise a miRNA, a circRNA, and a snoRNA.

### SUMMARY OF THE INVENTION

Against this background, the object underlying the invention was to address the challenges outlined above, in particular by providing new biomarkers which are specific for tumor cells or resp. tumor subtypes, and by providing alternative diagnostic methods utilizing lncRNA to detect and/or monitor a disease in a subject, in particular cancer. The method shall allow for the identification of subjects having disease conditions not detectable by conventional histopathological methods, and/or for the identification of subjects that benefit from a specific therapy, e.g., an oncogene-specific targeted drug, and/or for the monitoring of therapy success in the subject. Preferably, the method shall allow for the easy, reliable, sensitive, and fast detection of diagnostic and/or prognostic biomarkers, and more preferably shall be minimally invasive, especially without taking a tissue sample of the subject

The invention achieves this object by the features recited in the claims and further herein. In particular, the invention provides a method for detecting and/or monitoring a disease which is characterized by a specific oncogene activity, wherein the method comprises providing a serum/plasma sample from a subject and determining the expression level of one or more IncRNAs in the sample.

The term "disease" as used herein defines an illness which affects humans, in particular an illness involving abnormal cell growth such as a proliferative disorder or cancer. It is preferred that the disease is characterized by the activity of at least one specific oncogene, in particular Yes-associated protein (YAP) and/or Transcriptional Coactivator With PDZ-Binding Motif (TAZ; synonym: WW Domain-Containing Transcription Regulator Protein 1; WWTR1). The disease indicated by the method according to the invention preferably is a liver disease, e.g. selected from liver steatosis, non-alcoholic steatohepatitis, and liver cancer, e.g. hepatocellular carcinoma (HCC), in particular one in which at least one of the oncogenes YAP and TAZ is active due to dysregulation or overexpression. Therein, the disease can include all stages, e.g. all stages of cancer, including pre-malignant lesions. Moreover, the research leading up to the invention has revealed that next to liver cancer also other types of cancer can be detected by the method of the invention. Preferably the cancer is selected from liver cancer, e.g. HCC, and lung cancer, e.g. non-small-cell lung cancer (NSCLC), in particular lung adenocarcinoma. It is generally preferred that the disease is a subtype of HCC or NSCLC in which the oncogenes YAP and/or TAZ is/are active due to dysregulation or overexpression.

The inventors have found that expression levels of the one or more IncRNAs that are determined in the sample of a subject carrying the disease, which expression levels differ from the expression levels of the one or more IncRNAs determined in a negative control sample and/or in a sample of a healthy subject, and/or differ to a lesser degree from the expression levels determined in a positive control and/or in another subject carrying the disease, are indicative for the presence of the disease in the subject from which the analyzed sample was provided. In particular, it was found that the expression level of the one or more IncRNAs is/are indicative of the expression of YAP and/or the homologue protein TAZ, which are part of the Hippo pathway and which are controlling cell proliferation and apoptosis. An overexpression of the one or more IncRNAs in serum/plasma is therefore indicative for YAP and/or TAZ-activation in cells or tissue being present in the subject, which is in turn indicative for the disease being present in the subject. Consequently, in the method of the invention, an overexpression of the one or more IncRNAs in the sample indicates the presence of the disease in the subject the sample was taken from.

Moreover, the inventors have shown that the method can also be used to identify whether a subject would benefit from a future treatment, even if the subject has not previously been administered with the treatment Without being bound to this theory, this seems to be the case because the expression levels of the IncRNAs are indicative of whether a biological target for a drug is present in the subject, such that the method allows for predicting the therapeutic responsiveness for a specific drug.

Consequently, the invention further provides a method for identifying whether a subject would benefit from a future treatment, in particular wherein the subject has not previously been administered with the treatment the method comprising determining the expression level of one or more IncRNAs in a sample; wherein an overexpression of the one or more IncRNAs in the sample indicates that the subject will likely benefit from the treatment The term "likely to benefit" is used herein to mean that the treatment has a chance of > 50 % to lead to a positive outcome, e.g. indicated by a reduction in tumor growth and/or tumor size and/or reduced expression of the one or more IncRNAs.

Still further, the methods according to the invention allow for monitoring the disease by determining the expression level of one or more of the IncRNAs during and/or after treatment of the disease, preferably repeatedly. Therein, the method is particularly suitable to monitor the therapeutic success upon drug treatment, and/or to identify whether the disease relapses.

The features described herein for the method for detecting a disease in a subject can also be applied in any way to the method for identifying whether a subject would benefit from a future treatment, the method for identifying whether a treatment is successful or not, and the method for identifying whether a disease relapses, as all these methods pertain to the same inventive concept and underlying experimental findings.

According to the invention, the one or more IncRNAs is/are selected from the group consisting of CYTOR, SNHG17, MIR4435-2HG, SNHG1. The lncRNAs of the group are also described by the terms "signature IncRNAs", "lncRNA signature", "signature RNAs", and "RNA signature".

The IncRNAs to be detected each preferably have at least 90 % sequence identity, more preferably 95 % sequence identity, or 100 % sequence identity to one of the sequences as set out in Table 1 below:

**Table 1: Sequences of lncRNAs to be detected by the invention.**

| **IncRNA** | **IncRNA sequence ID** |
|---|---|
| CYTOR | SEQ ID No: 1 |
| MIR4435-2HG | SEQ ID No: 3 |
| SNHG1 | SEQ ID No: 4 |
| SNHG17 | SEQ ID No: 9 |

As usual in the field of IncRNAs, their sequences are provided in the enclosed sequence lisiting in the form of the corresponding DNA sequences. Further, the IncRNAs indicated herein by name also include corresponding variants such as splice variants or polymorphisms. Generally, each of the IncRNAs can be present in the sample in at least one splice variant For example, CYTOR can include at least one of the variants specified in SEQ ID No: 1-2, SNHG1 can include at least one of the variants specified in SEQ ID No: 4-8, and/or SNHG17 can include at least one of the variants specified in SEQ ID No: 9-22.

The term "expression level" as used herein refers to the concentration of an IncRNA in the sample. It has been found that the determination of the expression level of the IncRNAs according to the invention allows for the differentiation between healthy subjects and subjects carrying the disease to be analyzed, even if no clinically relevant condition is observed, i.e. even if the subject does not (yet) present any pathological disease symptoms.

The methods according to the invention allow for identifying, detecting and/or monitoring a disease, and for predicting whether a subject is likely to benefit from a future treatment, and for monitoring the therapeutic success upon drug treatment even when the expression level of only one of the IncRNAs is determined. However, the research leading up to the invention has shown that determining the expression level of more than one IncRNA increases the robustness of each of the methods. Consequently, the method preferably comprises determining the expression level of two or more, more preferably three or more, most preferably all four of the signature lncRNAs.

As generally preferred, the determining of the expression level of the one or more IncRNA comprises hybridization to at least one agent which is specific to detect the expression level of one of the lncRNAs, preferably with amplification by PCR. Therein preferably, the agents specific to detect the expression of one or more lncRNAs are nucleic acids comprising a nucleic acid sequence that shows at least 90% sequence identity to one of the sequences as set out in Table 2. It is especially preferred that the agents are present as primer pairs comprising a forward probe and a reverse probe, which primer pairs are suitable to amplify at least a fraction of one of the signature lncRNAs. The agents as set out in Table 2 are generally suitable to detect any of the splice variants mentioned above, such that an agent comprising or consisting of nucleic acid probes of SEQ ID No: 23 and 24 is suitable to detect CYTOR having a nucleic acid sequence of SEQ ID No: 1-2; an agent comprising or consisting of nucleic acid probes of SEQ ID No: 25 and 26 is suitable to detect SNHG17 having a nucleic acid sequence of SEQ ID No: 9-22; and an agent comprising or consisting of nucleic acid probes of SEQ ID No: 29 and 30 is suitable to detect SNHG1 having a nucleic acid sequence of SEQ ID No: 4-8.

**Table 2: Sequences of specific nucleic acid probes for the detection of lncRNAs.**

| **IncRNA** | **probe sequence and orientation** | **probe sequence ID** |
|---|---|---|
| CYTOR | forward probe: | SEQ ID No: 23 |
| | ATGCCCAAAGTTACGGAGGA | |
| | reverse probe: | SEQ ID No: 24 |
| | TATTCGAGGGATGCAGACGG | |
| SNHG17 | forward probe: | SEQ ID No: 25 |
| | AGCGTAGCTTCCTTGTCGTG | |
| | reverse probe: | SEQ ID No: 26 |
| | GAGACCTGACAGACAGCGTG | |
| MIR4435-2HG | forward probe: | SEQ ID No: 27 |
| | GTCATTAAGGTGGTCCTGCC | |
| | reverse probe: | SEQ ID No: 28 |
| | AGTGTCCTTTTCAGCGAGTGA | |
| SNHG1 | forward probe: | SEQ ID No: 29 |
| | ACGTTGGAACCGAAGAGAGC | |
| | reverse probe: | SEQ ID No: 30 |
| | GCAGCTGAATTCCCCAGGAT | |

The sample is generally preferably a liquid sample, more preferably a liquid biopsy sample obtained from a subject, e.g. from a patient presenting with a pathologic condition, or e.g., from a person having a pre-malignant or subpathologic disease condition or from a healthy person. Therein, the sample can comprise or, preferably, consist of any bodily fluid, including but not limited to blood, urine, stool, saliva, pleural effusions, cerebrospinal fluid or peritoneal fluid, or a cell-free fraction thereof. Preferably, the sample is whole blood or a fraction thereof, e.g., plasma or serum. Alternatively or additionally, the sample can comprise or consist of isolated tumor cells or tumor tissue material from the subject, e.g., a tumor biopsy, fresh or fixed, frozen and/or embedded in paraffine. In general, the term "liquid biopsy" means a blood sample that may contain malignant cells, e.g., tumor cells, or at least one IncRNA which is preferably released by tumor cells.

The research leading up to the invention has shown that the method according to the invention is unexpectedly sensitive in detecting a disease in a sample from a subject - so sensitive, in fact, that the method is suitable to detect a disease in a subject presenting no disease-specific symptoms, which subjects are also termed as having a sub-pathological disease condition. Currently, it is believed that the activation of the tumor-driving oncogenes YAP/TAZ is already detectable at early phases of tumorigenesis. Thus, the presence of the IncRNA should even be indicative for the disease in the earliest steps in tumorigenesis, where a solid tumor is not yet detectable, e.g., due to being too small to be detected by conventional tumor imaging methods.

The skilled person is well aware of how to determine expression levels of lncRNAs. In particular, the step of determining the expression level of an IncRNA can be carried out by reverse-transcribing the RNA contained in a sample containing the IncRNA to obtain cDNA, which is more stable in solution, optionally pre-amplifying, hybridizing to a specific nucleic acid probe, preferably a primer pair, amplifying, analyzing by semi-quantitative or quantitative PCR to generate a signal, which is preferably a fluorescence signal, and correlating the signal to an expression level of the IncRNA. As a general example, the signal determined can be the cycle threshold (ct) value of said IncRNA in a sample by quantitative PCR. Therein, generating cDNA by reverse transcription is advantageous because cDNA is generally more stable than IncRNA and can be used for amplifying methods such as real-time PCR.

The occurrence of an overexpression can be detected by comparing the IncRNA expression levels determined to a reference value, which in the following will be called "expression threshold". The expression threshold of an IncRNA can be the average expression level of said IncRNA in samples from undiseased subjects.

In general, an overexpression of the one or more IncRNAs in the sample indicates the disease. In particular, the disease can be indicated by discriminating the disease subtype from other subtypes of the same disease, e.g., discriminating a subtype of HCC having activated YAP and/or TAZ from a subtype of HCC without activated YAP and/or TAZ.

Accordingly, as used herein, the term "overexpression" generally refers to a determined expression level of the one or more IncRNAs which is/are above an expression threshold, such that the occurrence of an overexpression is generally preferably determined by comparing the expression level to the expression threshold. Therein preferably, the expression threshold is a predetermined value specific for the IncRNAs and/or the disease to be detected, e.g., determined from the mean expression of the IncRNAs in at least 1 sample from diseased subjects. Alternatively or additionally, the expression threshold can be determined from a plurality of reference samples obtained from healthy subjects, i.e. from subjects not having the disease.

It has been shown that more robust results can be achieved by the method if the expression levels of the one or more IncRNAs are normalized prior to determining whether an overexpression is present Consequently, the step of determining the expression level can additionally comprise the step of normalizing the expression level of each of the one or more IncRNAs. The normalizing preferably occurs by dividing the signal obtained for the IncRNA by the signal obtained for a spike-in control, or alternatively and less preferably by a signal obtained for a housekeeping gene, to generate a normalized signal, and correlating the normalized signal to an expression level to generate a normalized expression level of the one or more IncRNAs.

If a spike-in control is used to determine the expression level of an lncRNA, it is generally understood that the method prior to determining the expression level of the IncRNA, preferably immediately prior thereto, additionally comprises the step of adding a spike-in control to the sample. The spike-in control generally contains an artificial DNA/RNA oligonucleotide in a predetermined concentration. While choosing a suitable spike-in control is within the normal capabilities of the skilled person, the spike-in control can, by way of non-limiting example, have a sequence having at least 90 % sequence identity to SEQ ID No: 31, preferably a sequence identical to SEQ ID No: 31. Correspondingly, a nucleic acid probe or resp. primer pair suitable for detection of the spike-in control can have a sequence at least 90 % identical, preferably 100% identical to SEQ ID No: 32 and/or SEQ ID No: 33.

In an advantageous embodiment, the method further allows for an easy-to-interpret readout, which comprises only a single number, also referred to herein as a "score" or "signature score". The score bundles the expression values detected for each of the IncRNAs into a single value. While a score can be calculated even if only one IncRNA is detected, calculating the score is especially preferable if more than one IncRNA is detected in the sample. Consequently, in a preferred embodiment, the method can comprise the additional step of applying the expression level of each of the one or more IncRNAs into an algorithm to generate a score.

The signature score can be generated by an algorithm that processes the determined expression levels, preferably following normalization, of the one or more IncRNAs. In a preferred embodiment, the algorithm comprises, for each of the detected IncRNAs, generating a calibration curve containing the signal or normalized signal of the IncRNA and the signal determined from the spike-in control of known concentration or, less preferably, the housekeeping gene, to generate an expression level; normalizing the expression level by dividing by the mean value of the spike-in control to generate a normalized expression level; dividing the normalized expression level by the mean normalized expression level of said IncRNA determined in a plurality of reference samples which are taken from subjects with known disease state, e.g. from healthy subjects or from diseased subjects in a patient cohort, to generate a raw score value for each detected IncRNA. Subsequently, the algorithm generally comprises calculating the sum of the raw score values of each detected IncRNA to generate the signature score. Therein preferably, each detected IncRNA contributes to the signature score in equal weight The signature score is indicative of the abundance of the signature IncRNAs in the subject

In the same way that an overexpression is detected when the expression level of the one or more IncRNA is determined to be greater than the expression threshold, the signature score can be used to identify an overexpression by comparing to a score threshold. The score threshold is generally a predetermined value, e.g., predetermined for the combination of detected IncRNAs and/or for the disease to be detected. A score value that is determined to be above the score threshold indicates an overexpression. Therein, a score value of 0 is the lowest possible score value and corresponds to no signature IncRNA being present in the sample. The score value increases with the amount of signature IncRNA being present in the sample. In the experimental experience of the inventors, score values of up to 10 have been observed in samples containing particularly high amounts of IncRNA, but even higher values may be possible. By way of non-limiting example, a score value of below 1 can indicate that no disease is present in the subject and resp. that YAP and/or TAZ are not activated in the subject; a score value of 1 or above can indicate the presence of the disease in the subject or resp. indicates an overexpression of at least one of the signature IncRNAs, such that a score value of 1 would be the score threshold.

The score threshold can be determined from at least one control sample, preferably from a plurality of control samples. The at least one control sample can e.g. be obtained from a healthy subject. By way of example, if a signature of the four IncRNAs CYTOR, MIR4435-2HG, SNHG17, and SNHG1 is detected, the score threshold for indicating that HCC with YAP/TAZ activation is present in the subject can be 1, for NSCLC/lung adenocarcinoma with YAP/TAZ activity a score threshold can be 1. These scores must be evaluated for each disease separately using a high number of cancer patients and control samples.

The inventors have found that determining the score is also advantageous when evaluating whether the subject is likely to benefit from a specific treatment Drugs such as verteporfin or TED-347 have been applied to specifically inhibit YAP/TAZ activity. However, verteporfin is an FDA-approved photosensitizer and not suitable for the treatment of cancer patients. New drugs are currently developed and first publications suggest that novel drugs targeting YAP/TAZ will be available. As a consequence of the invention, it is now possible to assess whether a subject is likely to benefit from the specific YAP/TAZ-directed treatment without the need to acquire and analyse a tumor tissue sample. In detail, when a score value above the score threshold is determined or resp. if an overexpression of one or more of the signature IncRNAs is determined, the subject is likely to benefit from the future treatment

In any case, the method optionally further comprises the additional step of producing a report, wherein the report identifies the presence of the disease in the subject and/or the report identifies that the subject is likely to benefit from a future treatment if an overexpression of the one or more IncRNAs is detected, i.e. if the expression level of the one or more IncRNAs is/are greater than the expression threshold, e.g., if the score is greater than the score threshold. Moreover, in case that the report identifies the presence of the disease, the report can further identify that the subject is likely to benefit from a specific treatment if an overexpression is detected, e.g. if the score is greater than the score threshold.

The research preceding the invention has shown that the method is also suitable for monitoring a disease, especially monitoring the progression of a disease, by analyzing samples repeatedly taken from the same subject at different points in time. Therefore, the method can comprise the additional step of repeating the method at least once at a later point in time with a second, third, fourth etc. sample which stems from the same subject, preferably with generating a second, third, fourth etc. score. Advantageously, the later point in time is a point in time at least 1 week, 2 weeks, 3 weeks, 1 month, at least 2 months, at least 3 months, or preferably at least 6 months after the method has been carried out for the first time. Therein, a decrease of the score value over time indicates a regression of the disease, while an increase of the score value over time indicates a worsening of the disease.

In the spirit of the invention, the method is further suitable to determine whether an ongoing treatment of the disease is successful or not. The inventors have found that the amount of IncRNA expression decreases over time in the course of a successful treatment of the disease. Thus, when the method is repeated at a later point in time during an ongoing treatment of the disease, and the score value is determined to be lower than the score value determined the first time of performing the method, the treatment is successful; in contrast, if the score value is determined to be the same or higher than the score value determined the first time of performing the method, the treatment is not successful.

Still further, the method is suitable to determine whether a disease relapses or not. The research leading up to the invention has shown that after successful treatment of the disease and during subsequent monitoring of the disease, a new increase in the amount of IncRNA indicates that the disease relapses. It is to be expected that following a successful treatment, the signature score is below the score threshold. If, however, the method is repeated at a later point in time after finishing the treatment of the disease and the score value is determined to be above the score threshold, a relapse of the disease is indicated.

Taken together, calculating a score is advantageous because the attending physician can easily interpret whether a disease is present in the subject the sample was taken from, and/or whether the subject is likely to benefit from a specific future treatment, and/or whether treatment of the disease is successful, and/or whether a disease relapses.

In one embodiment, the method is for detecting a disease in a subject, the subject preferably having a non-pathological disease condition, the method comprising:
a. providing a liquid biopsy sample from a subject,
b. determining the expression level of one or more IncRNAs in the sample by contacting with a plurality of agents specific to detect the expression of the one or more IncRNAs, wherein the one or more lncRNAs is/are selected from SNHG1, MIR4435-2HG, SNHG17, and CYTOR, generally with reverse transcription of the IncRNAs prior to step b.,
c. optionally normalizing the expression level of each of the one or more IncRNAs to the expression level of a spike-in control to obtain normalized expression levels of each of the one or more IncRNAs,
d. applying each, optionally normalized, expression level into an algorithm to generate a score,
e. comparing the score to a predetermined score threshold, and
f. producing a report, wherein the report identifies the presence of the disease in the subject if the score is greater than the score threshold.

In a further embodiment, the method is for identifying whether a subject would benefit from a future treatment, the method comprising or consisting of the steps of:
a. providing a sample from a subject,
b. determining the expression level of one or more IncRNAs in the sample by contacting the sample with a plurality of agents specific to detect the expression of the one or more IncRNAs, wherein the one or more IncRNAs is/are selected from CYTOR, SNHG17, MIR4435-2HG, and SNHG1,
c. optionally normalizing the expression level of each of the one or more IncRNAs to the expression level of a spike-in control to obtain normalized expression levels of each of the one or more IncRNAs,
d. applying each, optionally normalized, expression level into an algorithm to generate a score,
e. comparing the score to a predetermined score threshold, and
f. producing a report, wherein the report identifies that the subject is likely to benefit from a future treatment if the score is equal to or greater than the score threshold.

In a still further embodiment, the method is for detecting whether a treatment is successful or not, the method, in addition to steps a. to f. as described above, comprising or consisting of the steps of:
g. repeating the method at least once at a later point in time using a different sample from the same subject to obtain a second score,
h. comparing the second score to the score obtained in step d. of the method, and
i. producing a report, wherein the report identifies that the treatment is successful if the second score is lower than the score; and wherein the report identifies that the treatment is not successful if the second score is equal to or higher than the score.

Similarly, in a still further embodiment, the method is for detecting whether a disease relapses, the method, in addition to steps a. to f. as described above, comprising or consisting of the steps of:
g. repeating the method at least once at a later point in time using a different sample from the same subject to obtain a second score,
h. comparing the second score to the score obtained in step d. of the method, and
i. producing a report, wherein the report identifies that the disease has relapsed if the second score is equal to or higher than the score threshold; and wherein the report identifies that the disease has not relapsed if the second score is below the score threshold,
wherein the score determined in step d. is below the score threshold.

The invention further relates to a diagnostic assay or kit, which is generally suitable for carrying out the method. For this purpose, the diagnostic assay generally comprises at least one spike-in control, e.g. a nucleic acid having a sequence of SEQ ID No: 31, and a plurality of agents specific to detect the expression of one or more IncRNAs, wherein the one or more IncRNAs is/are selected from the group comprising or consisting of CYTOR, SNHG17, MIR4435-2HG, SNHG1; and wherein the agents are nucleic acids having a nucleic acid sequence that shows at least 90% sequence identity to one of the sequences selected from SEQ ID No: 23-30, 32-33.

The features described for the methods of the invention also apply to the diagnostic assay or kit The diagnostic assay or kit is generally set up to carry out at least one of the methods of the invention.

The diagnostic assay or kit can optionally further comprise
- at least one receptacle for the sample,
- total RNA isolation means, e.g. suitable isolation buffers and elution columns,
- reverse transcription means, e.g. at least one buffer and at least one enzyme suitable for reverse transcription of isolated total RNA,
- pre-amplification means, e.g. at least one buffer suitable for pre-amplification of total RNA and/or reverse-transcribed nucleic acid using the plurality of agents specific to detect the expression of one or more IncRNAs, and/or
- a fluorescent dye that is set up to intercalate with DNA in a dose-dependent manner,
wherein the receptacle, total RNA isolation means, reverse transcription means, pre-amplification means, and fluorescent dye can be any which are known in the art.

The invention will now be described further with reference to the examples and to the figures, which show in
- Fig. 1 a random Forest-based Boruta analysis of lncRNAs combining data derived from HCC cell and publicly available human HCC patient data,
- Fig. 2 a statistical correlation between an IncRNA signature according to the invention and the known mRNA gene signature CIN25 in tissues of human HCC patients,
- Fig. 3 the expression level of the IncRNA signature in HCC tumor (T) and non-tumor (NT) tissues,
- Fig. 4A a random Forest-based Boruta analysis of lncRNAs combining data acquired in the context of LUAD from cell lines human cancer patients,
- Fig. 4B a statistical correlation between an IncRNA signature according to the invention and the known mRNA gene signature CIN25 in NSCLC patient data,
- Fig. 5 the expression level of the IncRNA signature in LUAD tumor (T) and non-tumor (NT) tissues,
- Fig. 6A individual expression levels of four IncRNAs in serum derived from healthy persons (NT) or HCC patients (T) of a first cohort,
- Fig. 6B individual expression levels of four IncRNAs in serum derived from healthy persons (NT) or HCC patients (T) of a second cohort,
- Fig. 6C the expression level of the IncRNA signature containing four IncRNAs in serum derived from healthy persons or HCC patients of the second cohort, and
- Fig. 7 a ROC analysis combining the first and second cohort for predicting the presence of nuclear YAP in HCC tissue samples.

### Example 1: Use of lncRNA signatures for assessing liver cancer

In a first study, those IncRNAs were identified which were reliably regulated by the oncogenes YAP and TAZ in HCC cells.

For this, gene expression data was obtained from the depmap (https://depmap.org/portal) cancer cell line encyclopedia and filtered for HCC cell lines with high YAP/TAZ activity. Gene expression values were z-scaled and visualized using the R packages pheatmap (https://CRAN.R-proiector.org/package=pheatmap) and ComplexHeatmap, respectively. YAP/TAZ were silenced in these cells and total RNA was subjected to next generation sequencing (NGS).

TCGA gene expression values from HCC patients were z-scaled and discretized into 10 bins using the R packages pheatmap and biclust (https://CRAN.R-project.org/package=biclust). K-mean clustering was applied to stratify patients into two groups (IncRNA high and IncRNA low) using the R package ComplexHeatmap. The Random Forest-based Boruta method according to Kursa, M. B., & Rudnicki, W. R., Journal of Statistical Software, 36(11), 1-13 (2010) was used to calculate the importance of each individual lncRNA for the assessment of patient condition.

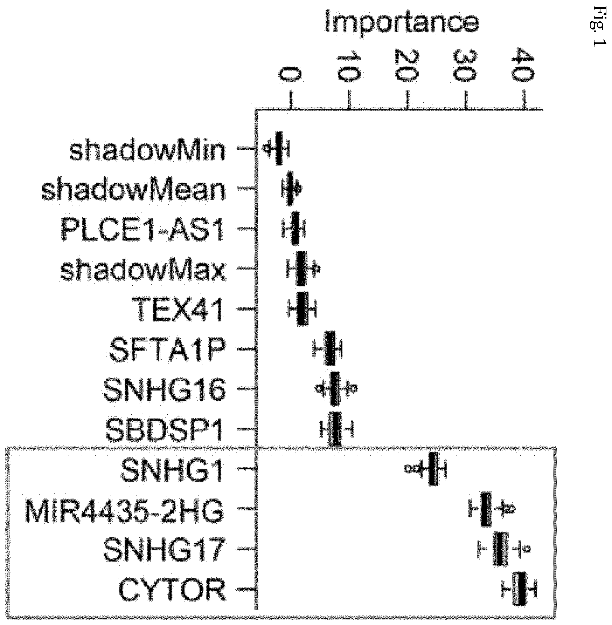

Fig. 1 shows the importance of each of the lncRNAs CYTOR, SNHG17, MIR4435-2HG, SNHG1, compared to the lncRNAs SBDSB1, SNHG16, SFTA1P, TEX41, and PLCE1-AS1 towards the classification of the HCC patient data. Data was acquired based on expression profiling. Additionally, three shadow features are shown that correspond to randomly shuffled features.

The experiment shows that the four lncRNAs CYTOR, SNHG17, MIR4435-2HG, and SNHG1 are more important for the classification of patients than the remaining five IncRNAs (Fig. 1, box).

From the expression values of these 4 lncRNAs, a signature score was calculated. To assure that each IncRNA contributes equally to the score, single expression values were divided by the mean expression of the corresponding IncRNA in all samples. For each patient, the calculated values were added. Balanced scores were statistically associated using the Spearman's rank correlation coefficient.

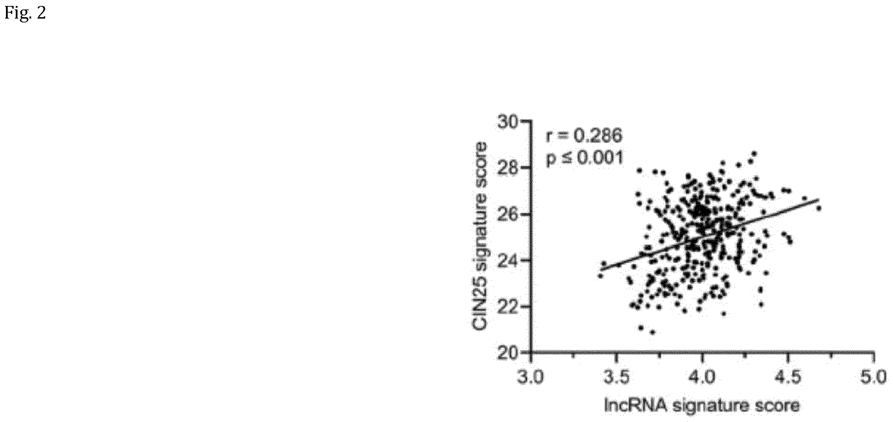

The IncRNA signature score in liver cancer tissues was compared to a previously known gene signature score, CIN25, which consists of YAP-regulated genes and which is used to measure chromosomal instability. CIN25 is e.g. known from Carter, S.L. et al., Nature genetics 38, 1043-1048 (2006). CIN25 detects mRNA molecules, which are highly unstable outside of cells and which cannot be detected as a gene signature or resp. genetic fingerprint. Moreover, the inventors have found that IncRNAs are released from tumor cells. The IncRNA signature score statistically correlated with the CIN25 signature score at a correlation coefficient of 0.286 (Fig. 2).

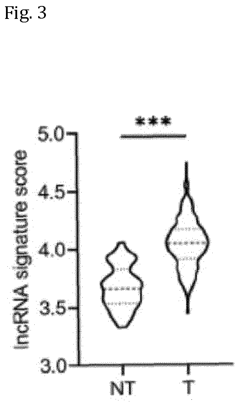

The IncRNA signature score was determined for human tumor tissue (Fig. 3: "T"), represented by HCC expression data, retrieved from the TCGA database, and for non-tumor tissue (Fig. 3: "NT"), represented by TCGA expression data of HCC patients and healthy liver specimens. The experiment shows that the IncRNA signature was significantly overexpressed in tumor tissues (Fig. 3), with p ≤ 0.001.

### Example 2: Use of lncRNA signature for assessing lung cancer

In a second study, similar to the one laid out in example 1 above, the importance of lncRNA expression in NSCLC (subtype LUAD) was evaluated. Data acquisition and processing was done as described above for Example 1. Different from Example 1, a LUAD cell lines was used for the genetic manipulation and subsequent NGS analysis.

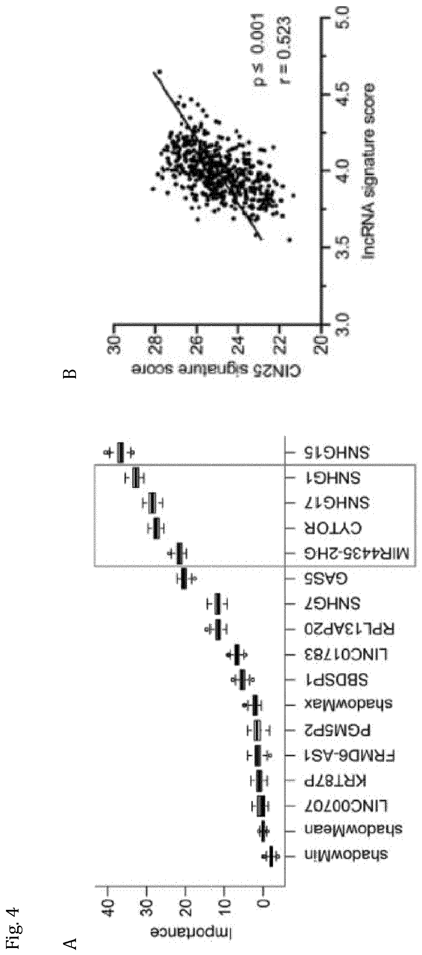

Surprisingly, it was found that the IncRNA signature constituents CYTOR, SNHG1, SNHG17, and MIR4435-2HG that had been identified for HCC cells were also reliable for classifying LUAD cells and LUAD patients with YAP/TAZ activation (Fig. 4A, Box). The IncRNA signature score statistically correlated with the CIN25 signature score at a correlation coefficient of 0.523 (Fig. 4B).

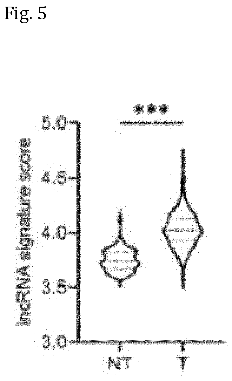

The presence of the IncRNA signature was higher in tumor tissue (Fig. 5 "T") than non-tumor tissue of LUAD patients (Fig. 5 "NT").

To substantiate the general relevance of the IncRNA signature according to the invention for the identification of tumors with YAP/TAZ activity, a comprehensive investigation of TCGA expression data comprising of 32 tumor types was performed including adrenocortical cancer (n=79), lung adenocarcinoma (n=497), head and neck cancer (n=494), and sarcoma (n=255). Interestingly, for many solid tumor entities, a significant correlation between the IncRNA signature (CYTOR, SNHG17, MIR4435-2HG, and SNHG1) and the YAP-regulated gene signature CIN25 was observed, ranging from a low (e.g., pancreatic cancer with rₛ=0.08, p=0.26) to moderate (head and neck cancer with rₛ=0.22, p≤0.001) to strong correlation (adrenocortical cancer with rₛ=0.50, p≤0.001).

### Example 3: lncRNA expression levels in the serum of liver cancer patients

Total RNA was isolated from serum samples using the miRNeasy Serum/Plasma Advanced Kit (Qiagen, Hilden, DE), according to the manufacturer's instruction. In brief, 200 µl serum were transferred into a 2 ml reaction tube. Subsequently, 60 µl RPL buffer were added, vortexed for 5 s and incubated at RT for 3 min. For sample normalization and to monitor variances in the RNA purification and amplification process, 3 µl of a custom spike-in control (10 nM in nuclease-free water) was added to the lysate. 20 µl RPP buffer were added to samples, vigorously vortexed for 20 s and incubated at RT for 3 min. To pellet the precipitated nucleic acid, samples were centrifuged at 12,000 x g at RT for 3 min. Subsequently, the supernatant was transferred to a new tube and 1 volume of isopropanol was added. The samples were loaded onto an Rneasy UCP MinElute spin column and centrifuged at 8,000 x g for 15 s. Samples were washed with 700 µl buffer RWT (8,000 x g for 15 s), 500 µl buffer RPE (8,000 x g for 15 s), and 500 µl 80 % ethanol (8,000 x g for 2 min). Columns were dried at full speed for 5 min and the RNA was eluted with 20 µl Rnase-free H₂O for 2 min.

Reverse transcription was performed with PrimeScript RT Master Mix (Takara Bio Inc., Kusatsu, JP) using 7 µl of total RNA according to the manufacturer's protocol. Subsequently, the IncRNA targets were pre-amplified using the Prelude PreAmp Master Mix. For this, equal volumes of each specific primer pair for candidate IncRNAs, negative control lncRNAs and spike-in control were added to each sample (final concentration: 500 nM). The pre-amplification reaction was performed for 14 cycles. Subsequently, the standard qPCR protocol, as previously described, was applied to quantify IncRNA expression levels. Detection of the spike-in control was used for normalization to account for any technical variances that occurred during the isolation process since house-keeping genes did not allow for reproducible sample normalization.

The expression levels of detected IncRNAs were normalized by comparing each individual signal to the signal of the spike-in control.

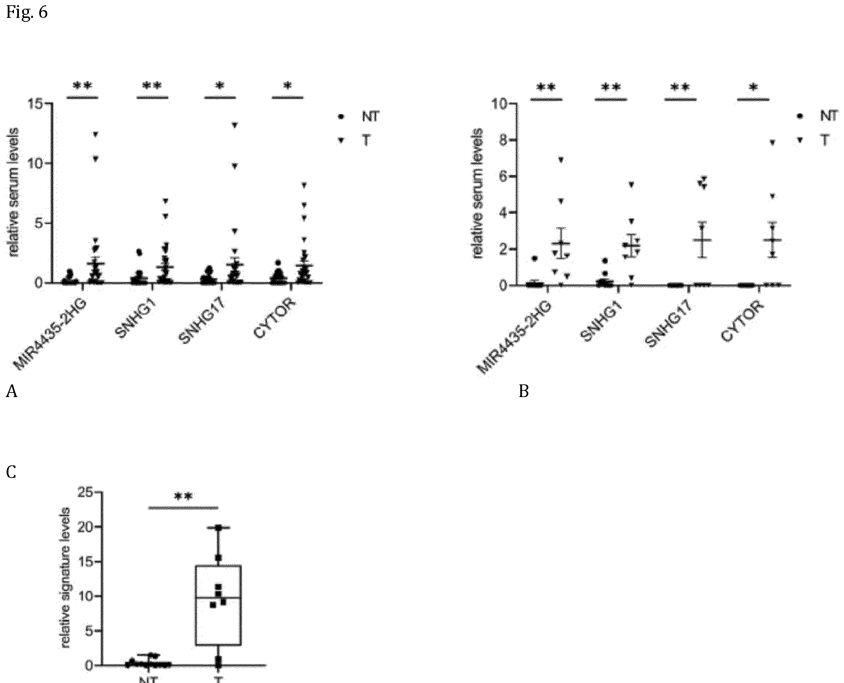

The individual expression levels of four IncRNAs, namely CYTOR, SNHG1, SNHG17, and MIR4435-2HG, were measured for a first cohort in serum derived from healthy persons (n=20; Fig 6A: "T") and HCC patients (n=29; Fig. 6A: "NT"). All lncRNAs were consistently detected in serum samples with elevated levels in a subtype of HCC patients compared to healthy controls (Fig. 6A).

For some (n=17) HCC patients of the first cohort, tissue samples were obtained, and immunohistochemistry stains for the oncogene YAP were carried out (data not shown). Correlating the relative abundance of the IncRNA signature with YAP positivity revealed a clear positive and significant statistical association (rₛ=0.75, p≤0.001].

For a second, confirmatory cohort of HCC patients (n=8), serum samples were obtained simultaneously with tissue samples. Expression levels of each of the four IncRNAs CYTOR, SNHG1, SNHG17, and MIR4435-2HG were measured in the serum of the HCC patients (Fig. 6B, "T), and compared to those measured in the serum of healthy persons (Fig. 6B, "NT"). Similarly, the expression level of the IncRNA signature containing all of the four IncRNAs was compared (Fig. 6C). In both cases, elevated expression of individual lncRNAs and the respective signature was detectable in a subgroup of patients (75% elevated, 25% not changed).

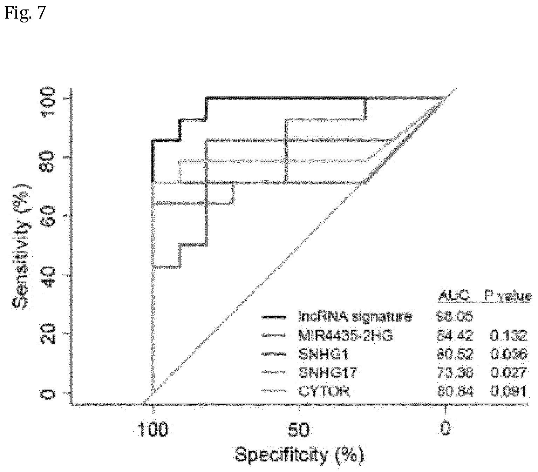

To define the predictive power of the IncRNA signature containing the four IncRNAs CYTOR, SNHG1, SNHG17, and MIR4435-2HG, the first and second cohort were combined and subjected to receiver operating characteristics (ROC) analysis. Indeed, the IncRNA signature better predicted the presence of nuclear YAP in HCC tissue samples than did individual lncRNAs (e.g., AUC_{sig}=98.05 vs. AUC_{SNHG17}=73.38). However, also each of the four IncRNA on its own could predict the presence of nuclear YAP in HCC tissue samples to a certain degree (Figure 7).

Together, these results illustrate that the method according to the invention can with high sensitivity and specificity identify cases with tumor-endogenous YAP activation by analysing a panel of lncRNAs in a sample, which preferably is a liquid biopsy sample like serum but can also be a solid sample such as tumor tissue.

## Claims

1. Method for identifying, detecting, and/or monitoring a disease, wherein the method comprises providing a sample from a subject and determining the expression level of one or more long non-coding RNAs (lncRNAs) in the sample;
wherein the one or more IncRNAs is/are selected from the group consisting of CYTOR, SNHG17, MIR4435-2HG, and SNHG1; and wherein an overexpression of the one or more long non-coding RNAs in the sample indicates the disease.

2. Method for identifying whether a subject would benefit from a future treatment, the method comprising providing a sample from a subject and determining the expression level of one or more long non-coding RNAs (lncRNAs) in the sample;
wherein the one or more IncRNAs is/are selected from the group consisting of CYTOR, SNHG17, MIR4435-2HG, and SNHG1; and wherein an overexpression of the one or more IncRNAs in the sample indicates that the subject is likely to benefit from the treatment

3. Method according to any one of the preceding claims, **characterized in that** the IncRNAs have at least 90 % sequence identity to the following sequences:
| | |
|---|---|
| CYTOR | SEQ ID No: 1 |
| MIR4435-2HG | SEQ ID No: 3 |
| SNHG1 | SEQ ID No: 4 |
| SNHG17 | SEQ ID No: 9 |

4. Method according to any one of the preceding claims, **characterized in that** the step of determining the expression level of one or more IncRNAs in a sample comprises determining the expression level of each of CYTOR, SNHG17, MIR4435-2HG, and SNHG1 in the sample.

5. Method according to any one of the preceding claims, **characterized in that** the sample is a liquid biopsy sample obtained from the subject.

6. Method according to any one of the preceding claims, **characterized in that** the disease is cancer, in particular selected from liver cancer and lung cancer, which has the activity of a specific oncogene.

7. Method according to any one of the preceding claims, **characterized in that** the step of determining the expression level additionally comprises normalizing the expression level of each of the one or more IncRNAs.

8. Method according to any one of the preceding claims, **characterized in that** prior to determining the expression level of the IncRNAs, the method additionally comprises the step of adding a spike-in control to the sample, which spike-in control contains a nucleotide molecule in a predetermined concentration.

9. Method according to any one of the preceding claims, **characterized in that** it comprises the additional step of applying the expression level of each of the one or more IncRNAs into an algorithm to generate a score, wherein the method further comprises comparing the score to a score threshold which is a predetermined value, e.g. predetermined for the disease to be detected, wherein a score that is determined to be above the score threshold indicates an overexpression.

10. Method according to any one of the preceding claims, **characterized in that** it further comprises the additional step of producing a report, wherein the report identifies the presence of the disease in the subject if the expression level of the one or more IncRNAs is/are equal to or greater than the expression threshold, or respectively if the score is equal to or greater than the score threshold.

11. Method according to any one of the preceding claims, **characterized in that** the method is repeated at least once at a later point in time with a different sample which stems from the same subject.

12. Method according to any one of the preceding claims, **characterized in that** the method prior to determining the expression level of the one or more IncRNAs comprises the step of reverse transcribing the IncRNAs in the sample to generate cDNA, followed by optional pre-amplification, wherein the determining the expression level is carried out with the resulting cDNA.

13. Diagnostic assay or kit comprising at least one spike-in IncRNA and
at least one agent specific to detect the expression of one or more IncRNAs,
wherein the one or more IncRNAs is/are selected from CYTOR, SNHG17, MIR4435-2HG, and SNHG1.

14. Diagnostic assay or kit according to claim 13, **characterized in that** the agents specific to detect the expression of one or more IncRNAs are nucleic acids comprising a nucleic acid sequence that shows at least 90% sequence identity to one of the following probe sequences:
| **IncRNA** | **probe sequence and orientation** | **probe sequence ID** |
|---|---|---|
| CYTOR | forward probe: | SEQ ID No: 23 |
| | ATGCCCAAAGTTACGGAGGA | |
| | reverse probe: | SEQ ID No: 24 |
| | TATTCGAGGGATGCAGACGG | |
| SNHG17 | forward probe: | SEQ ID No: 25 |
| | AGCGTAGCTTCCTTGTCGTG | |
| | reverse probe: | SEQ ID No: 26 |
| | GAGACCTGACAGACAGCGTG | |
| MIR4435-2HG | forward probe: | SEQ ID No: 27 |
| | GTCATTAAGGTGGTCCTGCC | |
| | reverse probe: | SEQ ID No: 28 |
| | AGTGTCCTTTTCAGCGAGTGA | |
| SNHG1 | forward probe: | SEQ ID No: 29 |
| | ACGTTGGAACCGAAGAGAGC | |
| | reverse probe: | SEQ ID No: 30 |
| | GCAGCTGAATTCCCCAGGAT | |
